Europäisches Patentamt

European Patent Office

Office européen des brevets

(11) Veröffentlichungsnummer: **0 241 716**
**B1**

# EUROPÄISCHE PATENTSCHRIFT

(45) Veröffentlichungstag der Patentschrift:
**29.08.90**

(21) Anmeldenummer: **87103628.1**

(22) Anmeldetag: **12.03.87**

(51) Int. Cl.⁵: **A61K 7/13**

(54) **Mittel und Verfahren zur oxidativen Färbung von Haaren.**

(30) Priorität: **27.03.86 DE 3610396**

(43) Veröffentlichungstag der Anmeldung:
**21.10.87 Patentblatt 87/43**

(45) Bekanntmachung des Hinweises auf die Patenterteilung:
**29.08.90 Patentblatt 90/35**

(84) Benannte Vertragsstaaten:
**AT BE CH DE ES FR GB GR IT LI LU NL SE**

(56) Entgegenhaltungen:
**EP-A- 0 043 436**
**EP-A- 0 166 100**
**DE-A- 3 314 565**
**FR-A- 2 315 255**
**FR-A- 2 421 869**

**Die Akte enthält technische Angaben, die nach dem
Eingang der Anmeldung eingereicht wurden und die
nicht in dieser Patentschrift enthalten sind.**

(73) Patentinhaber: **Wella Aktiengesellschaft, Berliner
Allee 65, D-6100 Darmstadt(DE)**

(72) Erfinder: **Konrad, Eugen, Mecklenburger Strasse 101,
D-6100 Darmstadt(DE)**
Erfinder: **Mager, Herbert Dr., Beaumont 5,
CH-1700 Fribourg(CH)**
Erfinder: **Hoffmann, Axel, An der Lettkaute 16,
D-6107 Reinheim 1(DE)**

**Beschreibung**

Gegenstand der Erfindung sind Mittel und ein Verfahren zur oxidativen Färbung von Haaren auf der Basis einer Kombination von Kuppler- und Entwicklersubstanzen, wobei 5-Amino-2-methyl-phenol als Kupplersubstanz und 4-Amino-3-methyl-phenol sowie 1,4-Diamino-benzol und/oder 2,5-Diamino-toluol als Entwicklersubstanzen verwendet werden.

Oxidationsfarbstoffe haben auf dem Gebiet der Haarfärbung eine wesentliche Bedeutung erlangt. Die Färbung entsteht hierbei durch die Reaktion bestimmter Kupplersubstanzen mit bestimmten Entwicklersubstanzen in Gegenwart eines geeigneten Oxidationsmittels.

An Oxidationsfarbstoffe, die zum Färben von menschlichen Haaren Verwendung finden, sind zahlreiche besondere Anforderungen gestellt. So müssen sie in toxikologischer und dermatologischer Hinsicht unbedenklich sein und die Erzielung von Färbungen in der gewünschten Intensität ermöglichen. Weiterhin ist es erforderlich, daß durch Kombination geeigneter Entwickler- und Kupplersubstanzen eine breite Palette unterschiedlicher Farbnuancen erzeugt werden kann. Außerdem wird für die erzielbaren Haarfärbungen eine gute Licht-, Dauerwell-, Säure- und Reibechtheit gefordert. Auf jeden Fall aber müssen solche Haarfärbungen ohne Einwirkung von Licht, chemischen Mitteln und Reibung über einen Zeitraum von mindestens 4 bis 6 Wochen stabil bleiben.

Als Entwicklersubstanzen werden vorzugsweise 1,4-Diamino-benzol, 2,5-Diamino-toluol, 2,5-Diamino-benzylalkohol sowie 4-Amino-phenol eingesetzt. 4-Amino-phenol dient vor allem zur Erzeugung von Modetönen, wobei in Kombination mit den Kupplersubstanzen 5-Amino-2-methyl-phenol ein Orange, mit 1-Naphthol ein violettstichiges Rot und mit m-Phenylendiamin und dessen Derivaten ein Violett entsteht. Von großem Nachteil ist hierbei, daß diese Farbtöne kein neutrales Rot darstellen sondern immer Blau- oder Gelbanteile enthalten. Ein weiterer bedeutender Nachteil ergibt sich beim Mischen dieser Farbtöne mit Naturtönen auf der Basis von 1,4-Diamino-benzol oder 2,5-Diamino-toluol. Hierbei wird nämlich nicht das erwartungsgemäße Farbergebnis erhalten, das zwischen dem Naturton und dem zugemischten Rotton liegt, sondern ein völlig aus dem Rahmen fallender violetter Farbton. Dieser violette Farbton wird durch die Reaktion des p-Diamins, beispielsweise des 1,4-Diamino-benzols, mit 1-Naphthol und mit m-Phenylendiamin oder dessen Derivaten verursacht.

In der DE-PS 1 143 605 werden Oxidationshaarfärbemittel beschrieben, welche als Kupplersubstanz 5-Amino-2-methyl-phenol in Kombination mit aromatischen 1,4-Diaminen enthalten. Diese Haarfärbemittel liefern jedoch nur violette Farbtöne, die wegen ihres blaustichigen Farbcharakters nur sehr begrenzt im Rottonbereich eingesetzt werden können.

Obwohl die Entwicklersubstanz 4-Amino-3-methyl-phenol in der Haarfärbeliteratur häufig erwähnt wird, hat sie keine oder nur eine geringe Bedeutung in der Färbepraxis erlangt. So liefert 4-Amino-3-methyl-phenol mit 5-Amino-2-methyl-phenol als Kuppler nur ein farbschwaches Ziegelrot.

Aufgabe der Erfindung ist es daher, ein Haarfärbemittel sowie ein Haarfärbeverfahren zur Verfügung zu stellen, bei dem die vorstehend genannten Nachteile vermieden werden.

Hierzu wurde nun überraschend gefunden, daß Mittel zur oxidativen Färbung von Haaren auf der Basis einer Kupplersubstanz/Entwicklersubstanz-Kombination, dadurch gekennzeichnet, daß sie als Kupplersubstanz

(A) 5-Amino-2-methyl-phenol
und als Entwicklersubstanzen
(B) 4-Amino-3-methyl-phenol und
(C) 1,4-Diamino-benzol und/oder 2,5-Diamino-toluol,

auch in Form der physiologisch verträglichen Salze, enthalten, in hervorragendem Maße der gestellten Aufgabe gerecht werden.

Als physiologisch verträgliche Salze mit anorganischen oder organischen Säuren kommen beispielsweise das Chlorid, Sulfat, Phosphat, Acetat, Propionat, Lactat oder Citrat in Betracht. Sofern die Färbesubstanzen aromatische OH-Gruppen besitzen, können sie auch in Form ihrer Salze mit Basen, beispielsweise als Alkaliphenolate, eingesetzt werden.

Während sich bei den Entwicklersubstanzen ein gewisser Überschuß an 4-Amino-3-methyl-phenol gegenüber 1,4-Diamino-benzol und/oder 2,5-Diamino-toluol als vorteilhaft erweist, kann die Kupplersubstanz 5-Amino-2-methyl-phenol in Bezug auf die Entwicklersubstanzen sowohl in äquimolarem Verhältnis als auch in einem Über- oder Unterschuß enthalten sein.

In den erfindungsgemäßen Haarfärbemitteln soll das 5-Amino-2-methyl-phenol in einer Menge von 0,1 bis 5,0 Gewichtsprozent, vorzugsweise 0,2 bis 3,5 Gewichtsprozent, enthalten sein.

Das 4-Amino-3-methyl-phenol soll in einer Menge von 0,1 bis 4,0 Gewichtsprozent, vorzugsweise 0,2 bis 3,0 Gewichtsprozent, enthalten sein, während das 1,4-Diamino-benzol und/oder das 2,5-Diamino-toluol in einer Gesamtmenge von 0,1 bis 2,0 Gewichtsprozent, vorzugsweise 0,2 bis 1,0 Gewichtsprozent, enthalten ist.

Mit der neuen Kupplersubstanz/Entwicklersubstanz-Kombination lassen sich neutrale rote Farbtöne, insbesondere Modetöne, die frei von Gelbanteilen und nahezu frei von Blauanteilen sind, erzielen.

Neben der hohen Farbintensität ist die hohe Leuchtkraft der erhaltenen Töne überraschend, die besonders bei der Färbung von pigmentiertem Naturhaar hervortritt. Ein weiterer Vorteil ist die hervorragende Farbegalität der Rottöne und das sehr gute Aufziehvermögen auf poröse Haarspitzen. Diesbezüglich ist die erfindungsgemäße Farbkombination Haarfärbemitteln mit roten Nitrofarbstoffen, zum Beispiel, 2-Nitro- 1,4-diamino-benzol, deutlich überlegen.

Die vorstehenden Ausführungen zeigen, daß die mit der erfindungsgemäßen Substanzkombination erzielbaren Haarfärbungen einen unerwarteten farbreinen Rotton ergeben, der nur bei gleichzeitiger Anwesenheit der zwei Entwicklersubstanzen (B) und (C) sowie 5-Amino-2-methyl-phenol (A) als Kupplersubstanz erzielt wird.

Das erfindungsgemäße System, bestehend aus der Kupplersubstanz (A) und den zwei Entwicklersubstanzen (B) und (C), ist sehr gut überschaubar und in der Färbepraxis leicht zu handhaben. Zudem werden die Substanzen der Komponenten (A), (B) und (C) großtechnisch hergestellt und stellen somit preiswerte Rohstoffe dar.

Weiterhin ist sowohl das 5-Amino-2-methyl-phenol als auch das 4-Amino-3-methyl-phenol in toxikologischer Hinsicht vorteilhaft. Durch die erfindungsgemäße Kupplersubstanz/Entwicklersubstanz-Kombination kann somit auf die physiologisch nicht völlig unbedenklichen Farbstoffe 4-Amino-phenol, 1-Naphthol, m-Phenylendiamin und die 2,4-Diamino-phenolether verzichtet werden.

Darüber hinaus ist mit der neuen Kupplersubstanz/Entwicklersubstanz-Kombination eine breite Palette verschiedener Modetöne, insbesondere ein hochmodisches Glutrot mit besonderer Leuchtkraft, zugänglich, welche mit 4-Amino-phenol als Entwickler nicht erzeugt werden können.

Jedoch sind die Färbemöglichkeiten nicht nur auf die Erzeugung intensiver, hochmodischer Farbtöne beschränkt. Der modische Farbcharakter läßt sich durch den Zusatz geeigneter Kupplersubstanzen als Nuanceure, beispielsweise der Kupplersubstanz 4-(2′-Hydroxyethyl)amino-1,2-methylendioxy-benzol, in Richtung eines natürlich wirkenden Farbtons verändern. Weiterhin können selbstverständlich neben der Komponente (A) auch übliche Kupplersubstanzen, beispielsweise 4-Amino-1,2-methylendioxy-benzol, 4-Hydroxy-1,2-methylendioxy-benzol, Resorcin, 4-Chlor-resorcin, 2-Methyl- resorcin, 3-Amino- phenol, Sesamol und 3,4-Methylendioxy-anilin in den neuen Haarfärbemitteln eingesetzt werden.

Für Nuancierungszwecke können neben den Komponenten (B) und (C) noch weitere bekannte Entwicklersubstanzen, beispielsweise 2,5-Diamino-benzylalkohol, zum Einsatz kommen.

Die bekannten Entwickler- und Kupplersubstanzen können in den Haarfärbemitteln jeweils einzeln oder im Gemisch miteinander enthalten sein.

Die Gesamtmenge der in den hier beschriebenen Haarfärbemitteln enthaltenen Kupplersubstanz/Entwicklersubstanz-Kombination soll etwa 0,3 bis 6,0 Gewichtsprozent, vorzugsweise 0,4 bis 5,0 Gewichtsprozent betragen.

Weiterhin können die Haarfärbemittel dieser Anmeldung zusätzlich andere Farbkomponenten, beispielsweise 6-Amino-2-methyl-phenol und 2-Amino-5-methyl-phenol, sowie ferner übliche direktziehende Farbstoffe, zum Beispiel Triphenylmethanfarbstoffe wie Diamond Fuchsine (C.I. 42 510) und Leather Ruby HF (C.I. 42 520), aromatische Nitrofarbstoffe wie 1,4-Diamino-2-nitro-benzol, 2-Amino-4-nitrophenol, 2-Amino-5-nitro-phenol und 2-Amino-4,6-dinitro-phenol, Azofarbstoffe wie Acid Brown 4 (C.I. 14 805) und Acid Blue 135 (C.I. 13 385), Anthrachinonfarbstoffe wie Disperse Red 15 (C.I. 60 710) und Disperse Violet 1 (C.I. 61 100), außerdem 1,4,5,8-Tetraamino-anthrachinon und 1,4-Diamino-anthrachinon enthalten.

Darüber hinaus können in den Haarfärbemitteln noch weitere übliche kosmetische Zusätze, beispielsweise Antioxidantien wie Ascorbinsäure, Resorcin oder Natriumsulfit, Parfümöle, Komplexbildner, Netzmittel, Emulgatoren, Verdicker, Pflegestoffe und andere vorhanden sein.

Die Zubereitungsform kann beispielsweise eine Lösung, insbesondere eine wäßrige oder wäßrig-alkoholische Lösung, sein. Die besonders bevorzugten Zubereitungsformen sind jedoch eine Creme, ein Gel oder eine Emulsion.

Ihre Zusammensetzung stellt eine Mischung der Farbstoffkomponenten mit den für solche Zubereitungen üblichen Bestandteilen dar.

Übliche Bestandteile von Lösungen, Cremes, Emulsionen oder Gelen sind zum Beispiel Lösungsmittel wie Wasser, niedere aliphatische Alkohole, beispielsweise Ethanol, Propanol, und Isopropanol, sowie mehrwertige Alkohole wie Ethylenglykol, 1,2-Propylenglykol und Glycerin, weiterhin Netzmittel oder Emulgatoren aus den Klassen der anionischen, kationischen, amphoteren oder nichtionogenen oberflächenaktiven Substanzen wie Fettalkoholsulfate, Alkylsulfonate, Alkylbenzolsulfonate, Fettsäuretauride, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Bentonit, Stärke, Polyacrylsäure, Cellulosederivate, Alginate, Vaseline, Paraffinöl und Fettsäuren sowie außerdem Pflegestoffe wie Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, während die Verdicker in einer Menge von etwa 0,1 bis 25 Gewichtsprozent in den Zubereitungen enthalten sein können.

Je nach Zusammensetzung können die erfindungsgemäßen Haarfärbemittel schwach sauer, neutral oder alkalisch reagieren. Insbesondere weisen sie einen pH-Wert im alkalischen Bereich zwischen 8,0 und 11,5 auf, wobei die Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid, Verwendung finden.

Die erfindungsgemäßen Haarfärbemittel auf der Basis der oben beschriebenen Kupplersubstanz/Entwicklersubstanz-Kombination ermöglichen Haarfärbungen von ausgezeichneter Egalität und Echtheitseigenschaften. Sie lassen sich mit Reduktionsmitteln wieder abziehen.

Hinsichtlich der färberischen Möglichkeiten bieten die erfindungsgemäßen Haarfärbemittel je nach Art und Zusammensetzung eine breite Palette verschiedener modischer und halbmodischer Farbnuancen, welche eine wesentliche Bereicherung und qualitative Verbesserung der bisher bekannten Färbemethoden darstellen.

Für die Anwendung zur oxidativen Färbung von Haaren vermischt man die vorstehend beschriebenen Haarfärbemittel unmittelbar vor dem Gebrauch mit einem Oxidationsmittel und trägt eine für die Haarbehandlung ausreichende Menge, je nach Haarfülle, im allgemeinen etwa 60 bis 200 g, dieses Gemisches auf das Haar auf. Als Oxidationsmittel zur Entwiclung der Haarfärbung kommen hauptsächlich Hydrogenperoxid oder dessen Additionsverbindungen an Harnstoff, Melamin oder Natriumborat in Form der 3 bis 12-prozentigen, wäßrigen Lösungen in Betracht. Wird eine 6-prozentige Hydrogenperoxidlösung als Oxidationsmittel verwendet, so beträgt das Gewichtsverhältnis zwischen Haarfärbemittel und Oxidationsmittel 5:1 bis 1:2, vorzugsweise jedoch 1:1. Größere Mengen an Oxidationsmittel werden vor allem bei höheren Farbstoffkonzentrationen im Haarfärbemittel oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet. Gegebenenfalls wird im Anschluß an diese Spülung mit einem Shampoo gewaschen und eventuell mit einer schwachen organischen Säure, wie zum Beispiel Zitronensäure oder Weinsäure nachgespült. Sodann wird das Haar getrocknet.

Durch die nachfolgenden Beispiele soll der Gegenstand der Erfindung näher erläutert werden.

| BEISPIELE | |
|---|---|
| Beispiel 1 | Haarfärbelösung |
| 3,0 g | 5-Amino-2-methyl-phenol |
| 2,0 g | 4-Amino-3-methyl-phenol |
| 0,3 g | 1,4-Diamino-benzol |
| 0,8 g | Natriumhydroxid, fest |
| 0,5 g | Natriumsulfit |
| 10,0 g | Isopropanol |
| 10,0 g | Laurylalkohol-diglykolethersulfat-Natriumsalz, 28-prozentige wäßrige Lösung |
| 10,0 g | Ammoniak, 25-prozentige wäßrige Lösung |
| 63,4 g | Wasser |
| 100,0 g | |

50 g des obigen Haarfärbemittels werden kurz vor dem Gebrauch mit 50 ml Hydrogenperoxidlösung (6-prozentig) vermischt und das Gemisch anschließend auf ergraute menschliche Haare aufgetragen. Nach einer Einwirkungszeit von 30 Minuten bei 40 Grad Celsius wird das Haar mit Wasser gespült und sodann getrocknet. Das Haar hat einen intensiven, neutralen Rotton angenommen, der frei von Gelb- und Violettanteilen ist.

| Beispiel 2 | Haarfärbemittel in Gelform |
|---|---|
| 1,5 g | 5-Amino-2-methyl-phenol |
| 0,9 g | 4-Amino-3-methyl-phenol |
| 0,6 g | 2,5-Diamino-toluol-sulfat |
| 0,4 g | Natriumhydroxid, fest |
| 0,6 g | Ascorbinsäure |
| 7,0 g | Isopropanol |
| 15,0 | Ölsäure |
| 10,0 g | Ammoniak, 25-prozentige wäßrige Lösung |
| 64,0 g | Wasser |
| 100,0 g | |

Man vermischt kurz vor dem Gebrauch 50 g dieses Haarfärbemittels mit 50 ml Hydrogenperoxidlösung (6-prozentig) und läßt das Gemisch 30 Minuten lang auf blonde menschliche Haare einwirken. Danach wird mit Wasser gespült und sodann getrocknet. Man erhält ein außergewöhnlich intensives, leuchtendes Glutrot.

| Beispiel 3 | Haarfärbecreme |
|---|---|
| 1,0 g | 5-Amino-2-methyl-phenol |
| 0,6 g | 4-Amino-3-methyl-phenol |
| 0,4 g | 2,5-Diamino-toluol-sulfat |
| 0,3 g | Natriumhydroxid, fest |
| 0,1 g | 4-(2'-Hydroxyethyl)amino-1,2-methylendioxy-benzol-hydrochlorid |
| 0,4 g | Natriumsulfit |
| 3,5 g | Laurylalkohol-diglykolethersulfat-Natriumsalz, 28-prozentige wäßrige Lösung |
| 15,0 g | Cetylalkohol |
| 10,0 g | Ammoniak, 25-prozentige wäßrige Lösung |
| 68,7 g | Wasser |
| 100,0 g | |

50 g des obigen Haarfärbemittels werden kurz vor dem Gebrauch mit 50 ml Hydrogenperoxidlösung (6-prozentig) vermischt und das Gemisch anschließend auf hellblondes, menschliches Haar aufgetragen. Nach einer Einwirkungszeit von 30 Minuten bei 40 Grad Celsius wird mit Wasser gespült und getrocknet. Das Haar ist n einem Mahagoniton gefärbt.

**Patentansprüche**

1. Mittel zur oxidativen Färbung von Haaren auf der Basis einer Kupplersubstanz/Entwicklersubstanz-Kombinaton, dadurch gekennzeichnet, daß es als Kupplersubstanz
(A) 5-Amino-2-methyl-phenol
und als Entwicklersubstanzen
(B) 4-Amino-3-methyl-phenol und
(C) 1,4-Diamino-benzol und/oder 2,5-Diamino-toluol,
auch in Form der physiologisch verträglichen Salze, enthält.

2. Mittel nach Anspruch 1, dadurch gekennzeichnet, daß das 5-Amino-2-methyl-phenol in einer Menge von 0,1 bis 5,0 Gewichtsprozent, vorzugsweise 0,2 bis 3,5 Gewichtsprozent, enthalten ist.

3. Mittel nach den Ansprüchen 1 und 2, dadurch gekennzeichnet, daß das 4-Amino-3-methyl-phenol in einer Menge von 0,1 bis 4,0 Gewichtsprozent, vorzugsweise 0,2 bis 3,0 Gewichtsprozent, enthalten ist.

4. Mittel nach den Ansprüchen 1 bis 3, dadurch gekennzeichnet, daß das 1,4-Diamino-benzol und/oder das 2,5-Diamino-toluol in einer Gesamtmenge von 0,1 bis 2,0 Gewichtsprozent, vorzugsweise 0,2 bis 1,0 Gewichtsprozent, enthalten ist.

5. Mittel nach den Ansprüchen 1 bis 4, dadurch gekennzeichnet, daß mindestens eine der Kupplersubstanzen 4-(2'-Hydroxyethyl)amino 1,2-methylendioxy-benzol, 4-Amino-1,2-methylendioxy-benzol, 4-Hydroxy-1,2-methylendioxy-benzol, Resorcin, 4-Chlor-resorcin, 2-Methyl-resorcin, 3-Amino-phenol, Sesamol und 3,4-Methylendioxy-anilin enthalten ist.

6. Mittel nach den Ansprüchen 1 bis 5, dadurch gekennzeichnet, daß die Gesamtmenge der Kupplersubstanz/Entwicklersubstanz-Kombination 0,3 bis 6,0 Gewichtsprozent, vorzugsweise 0,4 bis 5,0 Gewichtsprozent, beträgt.

7. Mittel nach den Ansprüchen 1 bis 6, dadurch gekennzeichnet, daß mindestens einer der direktziehenden Farbstoffe Diamond Fuchsine (C.I. 42 510), Leather Ruby HF (C.I. 42 520), 1,4-Diamino-2-nitro-benzol, 2-Amino-4-nitro-phenol, 2-Amino-5-nitro-phenol, Acid Brown 4 (C.I. 14 805), Acid Blue 135 (C.I. 13 385), Disperse Red 15 (C.I. 60 710), Disperse Violet 1 (C.I. 61 100), 1,4,5,8-Tetraamino-anthrachinon und 1,4-Diamino-anthrachinon enthalten ist.

8. Mittel nach den Ansprüchen 1 bis 7, dadurch gekennzeichnet, daß es Antioxidantien, vorzugsweise Natriumsulfit, Resorcin oder Ascorbinsäure, enthält.

9. Verfahren zur oxidativen Färbung von Haaren, dadurch gekennzeichnet, daß man ein Haarfärbemittel nach den Ansprüchen 1 bis 9 unmittelbar vor dem Gebrauch mit einem Oxidationsmittel, vorzugsweise Hydrogenperoxid oder dessen Additionsverbindungen an Harnstoff, Melamin oder Natriumborat, vermischt, sodann die Mischung in einer für die Haarbehandlung ausreichenden Menge auf das Haar aufträgt und nach einer Einwirkungszeit von etwa 10 bis 45 Minuten bei 15 bis 50 Grad Celsius das Haar mit Wasser spült, gegebenenfalls mit einem Shampoo wäscht, gegebenenfalls mit einem sauren Nachbehandlungsmittel nachspült und sodann trocknet.

## Claims

1. Composition for the oxidative dyeing of hair using as a base a coupler substance/developer substance combination, characterised in that it contains
   A) 5-amino-2-methyl-phenol as a coupler substance and
   B) 4-amino-3-methyl-phenol and
   C) 1,4-diamino-benzene and/or 2,5-diamino-toluol,
   as developer substances also in the form of physiologically compatible salts.

2. Composition according to claim 1, characterised in that the 5-amino-2-methyl-phenol is present in a quantity of 0.1 to 5.0% by weight and for preferece 0.2 to 3.5% by weight.

3. Composition according to claims 1 and 2, characterised in that the 4-amino-3-methyl-phenol is present in a quantity of 0.1 to 4.0% by weight and for preference 0.2 to 3.0% by weight.

4. Composition according to claims 1 to 3, characterised in that the 1,4-diamino-benzene and/or the 2.5-diamino-toluol is present in a total quantity of 0.1 to 2.0% by weight and for preference 0.2 to 1.0% by weight.

5. Composition according to claims 1 to 4, characterised in that at least one of the coupler substances 4-(2-hydroxyethyl)amino 1,2-methylene-dioxybenzene, 4-amino-1,2-methylene dioxybenzene, 4-hydroxy-1, 2-methylene dioxybenzene, resorcin, 4-chloro-resorcin, 2-methyl-resorcin, 3-amino-phenol, sesamol and 3,4-methylene dihydroxy-aniline.

6. Composition according to claims 1 to 5, characterised in that the total quantity of the coupler substance/developer substance combination is 0.3 to 6.0% by weight and for preference 0.4 to 5.0% by weight.

7. Composition according to claims 1 to 6, characterised in that it contains at least one of the direct-dyeing dyes Diamond Fuchsine (C.I. 42 510), Leather Ruby HF (C.I. 42 520), 1,4-diamino-2-nitro-benzene, 2-amino-4-nitro-phenol, 2-amino-5-nitro-phenol, Acid Brown 4 (C.I. 14 805), Acid Blue 135 (C.I. 13 385), Disperse Red 15 (C.I. 60 710), Disperse Violet I (C.I. 61 100), 1, 4, 5, 8-tetraamino-anthraquinone and 1,4-diamino-anthraquinone.

8. Composition according to claims 1 to 7, characterised in that it contains antioxidant agents, preferably sodium sulphite, resorcin or asorbic acid.

9. Process for the oxidative dyeing of hair, characterised in that a hair dye according to claims 1 to 9 is mixed immediately before use with an oxidizing agent, preferably hydrogen peroxide or its addition compounds of urea, melamine or sodium borate, after which the mixture is applied to the hair in a sufficient quantity for treating it, the hair is rinsed in water after a period of approximately 10 to 45 minutes at 15 to 50°C, where applicable washed with a shampoo and where applicable rinsed again in an acid aftertreatment composition and then dried.

## Revendications

1. Agent pour teindre les cheveux par oxydation, à base d'une combinaison de copulateur et de révélateur, caractérisé en ce qu'il contient comme copulateur:
   A) 5-amino–2–méthyl–phénol
   et comme révélateurs
   B) 4-amino–3–méthyl–phénol et
   C) 1,4-diamino–benzol et/ou 2,5-diamino–toluène,
   également sous la forme de sels physiologiquement compatibles.

2. Agent selon la revendication 1, caractérisé en ce que le 5-amino–2–méthyl–phénol est présent en une quantité comprise entre 0,1 et 5,0 pourcent en poids, de préférence entre 0,2 et 3,5 pourcent en poids.

3. Agent selon les revendications 1 et 2, caractérisé en ce que le 4-amino–3–méthyl–phénol est présent en une quantité comprise entre 0,1 et 4,0 pourcent en poids, de préférence entre 0,2 et 3,0 pourcent en poids.

4. Agent selon les revendications 1 à 3, caractérisé en ce que le 1,4-diamino–benzène et/ou le 2,5-diamino–toluène sont présents en une quantité totale de 0,1 à 2,0 pourcent en poids, de préférence de 0,2 à 1,0 pourcent en poids.

5. Agent selon les revendications 1 à 4, caractérisé en ce qu'au moins un des copulateurs est présent, à savoir 4-(2′-hydroxyéthyl)amino-1,2-méthylène-dioxy-benzène, 4-hydroxy-1,2-méthylène-dioxy-benzène, résorcine, 4-chlore-résorcine, 2-méthyl-résorcine, 3-amino-phénol, sésamol et 3,4-méthylène-dioxy-aniline.

6. Agent selon les revendications 1 à 5, caractérisé en ce que la quantité totale de la combinaison copulateur-révélateur est comprise entre 0,3 et 6,0 pourcent en poids, de préférence entre 0,4 et 5,0 pourcent en poids.

6

7. Agent selon les revendications 1 à 6, caractérisé en ce qu'au moins un des colorants qui montent directement est: fuchsine diamant (I.C 42 510), Leather Ruby HF (I.C 42 520), 1,4-diamino-2-nitro-benzène, 2-amino-4-nitrophénol, 2-amino-5-nitrophénol, brun acide 4 (I.C 14 805), bleu acide 135 (I.C. 13 385), Disperse Red 15 (I.C 60 710), Disperse Violet 1 (I.C 61 100), 1, 4, 5, 8-tétra–amino–anthraquinone et 1,4-diamino-anthraquinone.

8. Agent selon les revendications 1 à 7, caractérisé en ce qu'il contient des anti-oxydants, de préférence du sulfite de sodium, de la résorcine ou de l'acide ascorbique.

9. Procédé pour teindre les cheveux par oxydation, caractérisé en ce qu'on mélange une teinture pour cheveux selon les revendications 1 à 9 immédiatement avant l'utilisation, avec un agent d'oxydation de préférence de l'eau oxygénée ou ses produits d'addition avec urée, mélamine ou borate de sodium, puis le mélange est appliqué sur les cheveux en quantité suffisante pour le traitement, et, après avoir laissé agir d'environ 10 à 45 minutes à 15 à 50°C, on rince les cheveux à l'eau, on lave éventuellement avec un schampoing, on rince éventuellement avec un produit d'après-traitement acide et on sèche.